# EUROPEAN PATENT APPLICATION

(11) **EP 4 309 672 A1**
(43) Date of publication of application: **24.01.2024**
(21) Application number: 22770253.7
(22) Date of filing: 17.02.2022
(51) Int. Cl.: A61K 41/00, A61K 47/64, A61P 35/00

(54) **POLYPEPTIDE COUPLED BORON CARRYING AGENT, PREPARATION METHOD THEREFOR, AND PHARMACEUTICAL FORMULATION**

(30) Priority: 18.03.2021 CN 202110289774
(71) Applicant: Beijing University, Beijing 1000871 (CN)
(72) Inventor: XIANG, Jing, Beijing 1000871 (CN); REN, Qiushi, Beijing 1000871 (CN)
(74) Representative: Huang, Liwei
(86) International application number: PCT/CN2022/076659
(87) International publication number: WO 2022/193898

(57) **Abstract**

A boron carrying agent, a preparation method therefor, and a pharmaceutical formulation. The boron carrying agent contains compound 1, N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine, having a molecular formula of C₁₁₄H₁₅₈BN₃₀O₃₅S₁ and a molecular weight of 2552.4, and further contains compound 2, N-4-carboxyphenylboronic acid amide-arginine-lysine-lysine-lysine-arginine-arginine-glutamine-arginine-arginine-arginine, and/or compound 3, N-4-carboxyphenylboronic acid amide-methionine-alanine-serine-methionine-threonine-glycine-glycine-glutamine-glutamine-methionine-glycine. Compounds 1-3 can serve as a targeted medicament for boron neutron capture therapy used for treating a cerebral glioma and a head and neck tumor.

## Description

### Technical Field

The present invention relates to a polypeptide coupled boron carrying agent, a preparation method therefor, and a pharmaceutical formulation, in particular, to an N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine, a preparation method therefor, and a pharmaceutical formulation.

### Background Art

Tumors, especially malignant tumors, are diseases that seriously endanger human health in the world today. Its mortality rate is second only to cardiovascular disease, ranking second among the mortality rates of various diseases. In recent years, its morbidity has shown a significant upward trend. According to current cancer incidence trends, the number of new cancer patients worldwide will reach 15 million per year.

Glioma is a tumor that occurs in the neuroectoderm, and thus it is also named as neuroectodermal tumor or neuroepithelial tumor. Tumors originate from neurostromal cells, namely glia, ependyma, choroid plexus epithelium, and neural parenchymal cells, i.e., neurons. Most tumors originate from different types of glia. However, based on their histogenesis and similar biological characteristics, various tumors arising in the neuroectoderm are generally called gliomas.

There are many classification methods for glioma, and clinical workers often use the relatively simple Kemohan classification method. Among various types of gliomas, astrocytoma accounts for the most, followed by glioblastoma, and successively medulloblastoma, ependymoma, oligodendroglioma, pineal tumor, mixed glioma, choroid plexus papillomas, unclassified gliomas, and neuronal tumors. The predilection sites of various types of gliomas are different. For example, astrocytomas are more common in the cerebral hemispheres in adults and in the cerebellum in children; glioblastomas almost always occur in the cerebral hemispheres; medulloblastomas occur in the cerebellar vermis; and ependymomas are more common in the 4th ventricle.

At present, the treatment methods for glioma are classified into surgery, radiotherapy and chemotherapy according to the effect. Since gliomas grow invasively and have unclear boundaries with surrounding tissues, even if a microscope is used during surgery, a small amount of tumor cells will inevitably be left over, leading to tumor recurrence. Although new progress has been made in chemotherapy, it is difficult for systemic chemotherapy to pass through the blood-brain barrier and achieve an effective drug concentration in the local area of the tumor. Moreover, high-dose chemotherapy causes obvious systemic toxic and side effects, such as bone marrow suppression, reduction of white blood cell and platelet, pulmonary fibrosis, etc. Although each treatment modality has made significant progress, and the combined therapy of the three methods can prolong the patient's survival, glioma is still difficult to be cured. In recent years, new therapies such as boron neutron capture therapy have been adapted from the laboratory to the clinic.

Boron neutron capture therapy (BNCT) is a new method for treating tumors. It was originally proposed by Locher from the United States in 1936. A boron (¹⁰B) carrier agent having specific affinity for tumors is injected into human body, and accumulated in tumor cells, followed by irradiation with super-heated low-energy neutrons (1eV∼10keV), ¹⁰B nuclei reacts with neutrons, and the α particles and ⁷Li³⁺ produced will kill tumor cells: ¹⁰B+¹n→[¹¹B]→⁴He²⁺ (α)+⁷Li³⁺+2.31 MeV. Studies have shown that the neutron reaction cross-section of ¹⁰B in tumor cells is 3800 b (barn), and neutron capture reactions can also occur with hydrogen (¹H) and nitrogen (¹⁴N) in normal cells around the tumor. But the neutron capture reaction cross-sections of ¹H and ¹⁴N are only 0.332 b and 1.82 b, respectively. Thus, compared to the tissue around the tumor, the ¹⁰B reaction cross-section to neutrons in the tumor cells is large and the toxic and side effects are relatively low. However, it was not until the 1980s that major breakthroughs were made in the preparation of boron compound carriers and the problem of thermal neutron sources was simultaneously solved, allowing BNCT to enter the clinical practical stage. Clinically, BNCT is mainly used for treating cancers such as cerebral tumors, gliomas, and melanomas.

The boron carrying agents used in BNCT are a series of boron-containing compounds being passive or active in targeting whose molecules can be selectively taken up by tumor cells. The ideal boron carrying agent should have the following features: 1) The boron dose must meet the requirements, at least ensuring that each gram of tumor tissue absorbs about 10-30 µg¹⁰B, that is, each tumor cell contains 10^{9 10}B atoms, and during the irradiation period, the radiation dose of boron within the tumor cells should be kept above 1 Gy. 2) The ratio of the boron concentration of tumor cells to normal tissue and the ratio of boron concentration of tumor cells to blood must be guaranteed to be equal to or above 3 to ensure the targeting performance of the boron carrying agent. 3) In addition to being taken up by tumor cells at the dividing stage, it can also be taken up by tumor cells. 4) It is non-toxic and has favorable water solubility.

The second generation BNCT reagents are a series of boron-containing organic small molecule compounds being passive in targeting, whose molecules can be selectively absorbed by tumor cells. In terms of clinical treatment, there are two boron-containing drugs approved by the US FDA:
1) Sodium mercaptododecaborate (BSH: Na₂B₁₂H₁₁SH): BSH is a water-soluble compound containing 12 B atoms in the molecule. Experience has proven that BSH has the best aggregation effect in the treatment of cerebral tumors. It was originally synthesized by Miller et al. in 1964 and was first used for the treatment of cerebral tumors in 1968. BNCT irradiation was adopted during craniotomy and achieved favorable therapeutic effect. In 2018, Goeun Choi et al. combined layered double hydroxide (LDH) with BSH. After U87 cells absorbed BSH-LDH nanoparticles, the ¹⁰B content was 42.4 µg/10⁶ cells, which was approximately 2000 times larger than the minimum boron requirement of BNCT. The T/B value of ¹⁰B in mice of the BSH-LDH group was also 4.4 times higher than that of the BSH group within 2 hours. Excellent neutron capture efficiency was also finally produced under the condition of 30 µg ¹⁰B mL⁻¹.
2) ¹⁰B-p-Dicarboxylboronic acid phenylalanine (BPA: ¹⁰BC₉H₁₂NO₄). BPA is a liposoluble compound with a structure similar to phenylalanine. Since tumor cells have strong metabolism and require more phenylalanine than normal cells, tumor cells can actively take up large amounts of BPA. However, because its structure contains boron, BPA is not involved in protein synthesis. Based on the hypothesis that BPA is preferentially taken up by melanin-synthesizing cells, it was initially administered intravenously to treat patients with cutaneous melanoma. Experiments by Coderre et al. in 1990 showed that BPA was also taken up by other types of tumor cells, including rat cerebral 9L gliosarcoma. Mallesh et al. found that the fructose complex of BPA (BPA-F) can significantly improve its water solubility and increase its solubility in the blood. It can be injected intravenously. Therefore, BPA quickly entered the clinical stage and is used for treating patients with malignant melanoma and cerebral glioma.

Due to defects at the level of molecular property, BPA and BSH are not specific enough for tumor cells and cannot meet the aforementioned ideal standards for boron carrying agent. The major challenges in the development of boron carrying agents are the need to selectively target tumor cells and deliver therapeutic concentrations of boron with minimal normal tissue uptake and retention. Directions to improve the selectivity of boron carrying agents include integration with tumor-targeting groups, such as unnatural amino acids, polyamines, polypeptides, proteins, antibodies, nucleosides, sugars, porphyrins, liposomes, and nanoparticles.

One of the main indications for boron neutron capture therapy is cerebral glioma and head and neck tumor. Drugs need to penetrate the blood-brain barrier to reach cerebral gliomas. Thus, subsequent research on boron carrying agents needs to transport sufficient doses of ¹⁰B molecules into tumor cells and maintain a relatively high T/N value. Among them, effectively killing glioma cells without destroying normal brain tissue is more challenging than the treatment of malignant tumors in other locations. The blood-brain barrier blocks drugs with molecular weight greater than 200 Da, and the highly infiltrative nature of glioma cells and their genomic heterogeneity make it more difficult to treat cerebral gliomas.

### Summary

One object of the present invention is to provide a polypeptide boron carrying agent and a pharmaceutically acceptable salt thereof.

The boron carrying agent according to the present invention is an N-4-carboxyphenylboronic acid amide polypeptide compound. The compound has the advantages of stable quality and excellent properties, and is more suitable as a raw material for preparing various forms of pharmaceutical preparations.

The technical solutions of the present invention for achieving the above object are:
The boron carrying agent and a pharmaceutically acceptable salt thereof according to the present invention is N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine and a pharmaceutically acceptable salt thereof, the specific molecular formula is:
The N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine compounds (ANG) according to the present invention have a molecular formula of C₁₁₄H₁₅₈BN₃₀O₃₅S₁ and a molecular weight of 2552.4. This is compound 1.

The chemical name of compound 2 is N-4-carboxyphenylboronic acid amide-arginine-lysine-lysine-lysine-arginine-arginine-glutamine-arginine-arginine-arginine (TAT).

The chemical name of compound 3 is N-4-carboxyphenylboronic acid amide-methionine-alanine-serine-methionine-threonine-glycine-glycine-glutamine-glutamine-methionine-glycine (T7).

Another object of the present invention is to provide a pharmaceutical preparation, which comprises: (1) the boron carrying agent according to the present invention and/or a pharmaceutically acceptable salt thereof; and (2) one or more pharmaceutically acceptable carriers or excipients.

The compound or pharmaceutical preparation according to the present invention can be used to prepare a targeted medicament for boron neutron capture therapy for treating a cerebral glioma and a head and neck tumor.

A further object of the present invention is to provide a preparation method for the above-mentioned compound, comprising the following steps:
1. A first amino acid was coupled to 4-carboxyphenylboronic acid. The NMHA resin was swollen in DCM for 30 minutes in advance, suction filtered and rinsed with DMF. A solution of threonine and DIPEA in dichloromethane was added to the resin. The resin mixture was reacted with stirring for 2 hours.
2. Suction filtration. The resin was treated with a mixture of DCM/DIPEA/MeOH (17:1:2), and then rinse with DMF (3×30 seconds), methanol (3×30 seconds) and DCM (3×30 seconds).
3. The remaining amino acids are coupled according to the conventional Fmoc synthesis method.
4. DMF solution containing 20% piperidine was used for treatment for 10 minutes. DMF, CH₂Cl₂ and DMF were successively used for rinsing and coupling. 4 mL HBTU, 2 mL HOBT and 206.58 µL DIPEA were added to 5 ml of Fmoc amino acid solution successively, and then 700 µL DMF was added for dissolving. The above mixture was added to the resin mixture. After reacting for 1 hour, DMF, CH₂Cl₂ (2x) and DMF were successively used for washing;
5. HPLC high performance liquid chromatography was used to purify and separate the synthesized polypeptides. Conditions: C18 chromatographic column ODS-34.6×250 mm, mobile phase A: H₂O (0.065% trifluoroacetic acid), mobile phase B: acetonitrile (0.05% trifluoroacetic acid), flow rate 1 mL/min, gradient conditions: A: 0-25 min, 5%-65%; 25-27 min, 65%-95%; 27-35 min, 95%-5%.

The present invention has the following advantages:
The present invention is directed to a targeted medicament for boron neutron capture therapy, and one of its main indications is cerebral glioma and head and neck tumor. Since gliomas grow aggressively and have unclear boundaries with surrounding tissues, a small amount of tumor cells will inevitably be left over in surgery, leading to tumor recurrence. Although new progress has been made in chemotherapy, it is difficult for systemic chemotherapy to pass through the blood-brain barrier and achieve an effective drug concentration in the local area of the tumor. Moreover, high-dose chemotherapy causes obvious systemic toxic and side effects. The present invention has prospect to effectively kill glioma cells without damaging normal brain tissue, reduce damage to the brain, and has wide applications.

### Brief Description of Drawings

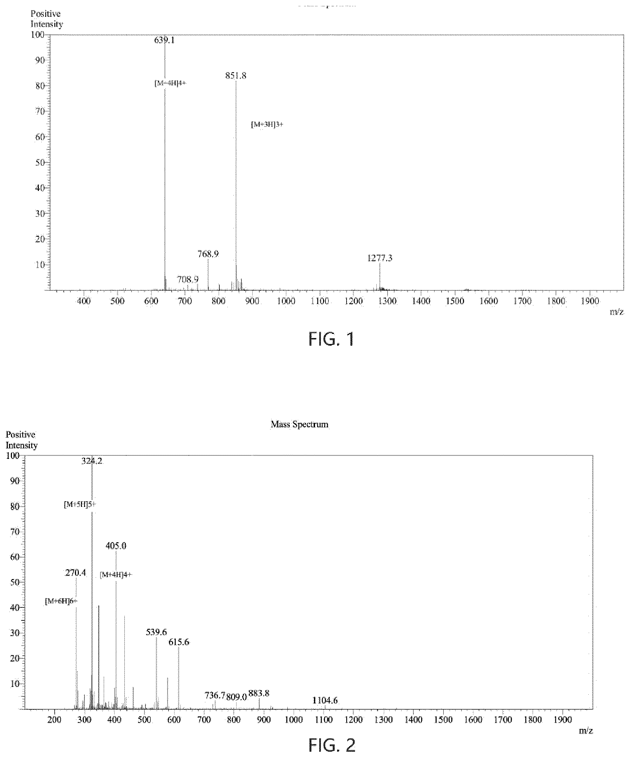

FIG. 1 is a mass spectrum of the N-4-carboxyphenylboronic acid amide polypeptide compound prepared in Example 1 of the present invention.
FIG. 2 is a mass spectrum of N-4-carboxyphenylboronic acid amide-arginine-lysine-lysine-lysine-arginine-arginine-glutamine-arginine-arginine-arginine.
FIG. 3 is a mass spectrum of N-4-carboxyphenylboronic acid amide-methionine-alanine-serine-methionine-threonine-glycine-glycine-glutamine-glutamine-methionine-glycine.
FIG. 4 shows the uptake of the present product and BPA in different cells within 12 hours.
FIG. 5 shows the uptate of polypeptides in different tissues.

### Detailed Description of Embodiments

The present invention will be described in further detail below in combination with specific embodiments. The purpose of the embodiments is only to illustrate and describe the current best embodiments of the present invention. The scope of the present invention is not limited in any way by the embodiments described herein.

### Example 1

Preparation of N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine

A first amino acid was coupled to 4-carboxyphenylboronic acid. The NMHA resin was swollen in DCM for 30 minutes in advance, suction filtered and rinsed with DMF. A solution of threonine and DIPEA in dichloromethane was added to the resulting resin to form a resin mixture. The resin mixture was reacted with stirring for 2 hours. Suction filtration. The resin was treated with a mixture of DCM/DIPEA/MeOH (17:1:2, in volume), and then rinse with DMF (3×30 seconds), methanol (3×30 seconds) and DCM (3×30 seconds).

### Example 2

Purification of N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine

DMF solution containing 20% piperidine was used for treatment for 10 minutes. DMF, CH₂Cl₂ and DMF were successively used for rinsing and coupling. 4 mL HBTU (189.62 mg), 2 mL HOBT (33.78 mg) and 206.58 µL DIPEA were added to 5 ml of Fmoc amino acid solution successively, and then 700 µL DMF was added for dissolving. The above mixture was added to the resin mixture. After reacting for 1 hour, DMF, CH₂Cl₂ (2x) and DMF were successively used for washing.

HPLC high performance liquid chromatography was used to purify and separate the synthesized polypeptides, with conditions: C18 chromatographic column ODS-34.6×250 mm, mobile phase A: H₂O (0.065% trifluoroacetic acid), mobile phase B: acetonitrile (0.05% trifluoroacetic acid), flow rate 1 mL/min, gradient conditions: A: 0-25 min, 5%-65%; 25-27 min, 65%-95%; 27-35 min, 95%-5%.

### Example 3

### In vitro study on the compound according to the present invention

N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine, N-4-carboxyphenylboronic acid amide-arginine-lysine-lysine-lysine-arginine-arginine-glutamine-arginine-arginine-arginine and N-4-carboxyphenylboronic acid amide-methionine-alanine-serine-methionine-threonine-glycine-glycine-glutamine-glutamine-methionine-glycine were formulated into aqueous solutions of 0.1 mg/g, 0.2 mg/g, 0.4 mg/g, 0.6 mg/g and 0.8 mg/g, added to U87MG, U251, A375, A549, HS683 and Hep cell lines cultured in 25T petri dishes, respectively, and incubated in a thermostatic incubator for 12 hours. Subsequently, the cells and culture medium were separated, and the number of cells was determined. After digesting the cells with concentrated nitric acid, the B content in the cells was determined by ICP-AES.

Meanwhile, the BPA was formulated into aqueous solutions of 0.1 mg/g, 0.2 mg/g, 0.4 mg/g, 0.6 mg/g, and 0.8 mg/g, added into U87MG, U251, A375, A549, HS683 and Hep cell lines cultured in 25T petri dishes, respectively, and incubated in a thermostatic incubator for 12 hours. Subsequently, the cells and culture medium were separated, and the number of cells was determined. After digesting the cells with concentrated nitric acid, the B content in the cells was determined by ICP-AES.

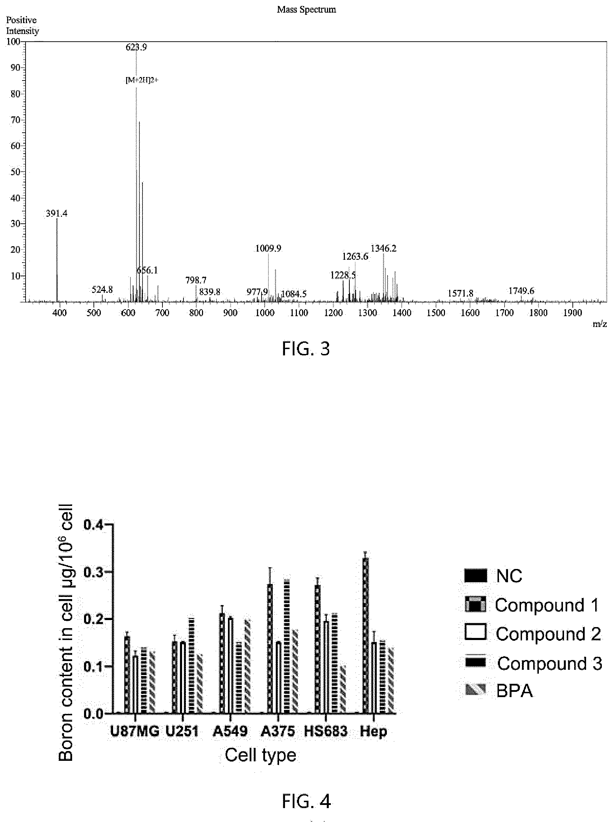

The detection limit is 249.772 nm. After determination, the B content contained in every 10⁶ cells can be calculated and plotted in FIG. 4. As can be seen, within 12 hours, the boron concentration in the cells is positively correlated with the concentration of the added product, with increasing trend gradually slowing down. Within 12 hours, best uptate of the products was observed in the glioma cell line U251.

Comparison shows that in term of uptate in cerebral glioma HS683 and A375 cells, both compound 1 and compound 2 were significantly higher than BPA.

### Example 4

### In vivo study on the compound according to the present invention

Healthy BALB/c mice with an average weight of approximately 21 g were selected and inoculated with HS683 cerebral glioma to establish a mouse cerebral glioma model. After 21 days, the mouse cerebral glioma model was generated, and 500 mg/kg of BPA, N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine, N-4-carboxyphenylboronic acid amide-arginine-lysine-lysine-lysine-arginine-arginine-glutamine-arginine-arginine-arginine and N-4-carboxyphenylboronic acid amide-methionine-alanine-serine-methionine-threonine-glycine-glycine-glutamine-glutamine-methionine-glycine solutions were injected through the tail vein of the mice. After administration, the major tissues and organs (heart, liver, spleen, lungs, kidneys and tumor sites) of the mice were removed at selected time periods (1 hour, 2 hours, and 4 hours). After washing and weighing, the tissues and organs were digested with concentrated nitric acid. The digested tissue sample was diluted and the concentration in the sample was determined by ICP-MS.

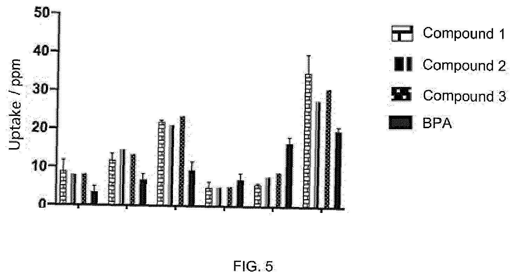

As shown in FIG. 5, two hours after administration, it can be seen that in the mouse cerebral glioma site, the uptake of the three compounds is higher than that of BPA, and the uptake of compound 1 is the highest, indicating that this product can improve the targeting effect of boron carrying agent and increase the effective neutron absorption during BNCT irradiation.

The specific embodiments of the present invention have been described above, however, it should be understood that the present invention is not limited to the above-mentioned specific embodiments. Those skilled in the art can make various modifications and changes, which all fall into the protection scope of the present invention, without departing from the concept of the present invention.

## Claims

1. A boron carrying agent selected from:
compound 1, wherein the compound 1 is N-4-carboxyphenylboronic acid amide-threonine-phenylalanine-phenylalanine-tyrosine-glycine-glycine-serine-arginine-glycine-lysine-arginine-aspartic acid-aspartic acid-phenylalanine-lysine-threonine-glutamic acid-glutamic acid-tyrosine-cysteine;
compound 2, wherein the compound 2 is N-4-carboxyphenylboronic acid amide-arginine-lysine-lysine-lysine-arginine-arginine-glutamine-arginine-arginine-arginine; and
compound 3, wherein the compound 3 is N-4-carboxyphenylboronic acid amide-methionine-alanine-serine-methionine-threonine-glycine-glycine-glutamine-glutamine-methionine-glycine.

2. The boron carrying agent according to claim 1, wherein the compound 1 has a molecular formula of C₁₁₄H₁₅₈BN₃₀O₃₅S₁ and a molecular weight of 2552.4.

3. A pharmaceutical formulation comprising:
(1) the boron carrying agent according to claim 1, and/or a pharmaceutically acceptable salt thereof; and
(2) one or more pharmaceutically acceptable carriers or excipients.

4. Use of any one of claims 1-3 in the preparation of a targeted medicament for a boron neutron capture therapy.

5. The use according to claim 4, wherein the targeted medicament for a boron neutron capture therapy is used for treating a cerebral glioma and a head and neck tumor.

6. A method for preparing the boron carrying agent according to claim 1, comprising the following steps:
(1) coupling a first amino acid to 4-carboxyphenylboronic acid; swelling the MBHA resin in DCM for 30 minutes in advance, followed by suction filtration and rinsing with DMF; adding a solution of threonine and DIPEA in dichloromethane to the resulting resin; and allowing the resin mixture to react with stirring for 2 hours;
(2) suction filtering; treating the resin with a mixture of DCM/DIPEA/MeOH with 17:1:2 in volume, and then rinsing with DMF in 3×30 seconds, methanol in 3×30 seconds and DCM in 3×30 seconds;
(3) coupling the remaining amino acids according to the conventional Fmoc synthesis method;
(4) treating with DMF solution containing 20% piperidine for 10 minutes; rinsing with DMF, CH₂Cl₂ and DMF successively, and coupling; adding 4 mL HBTU, 2 mL HOBT and 206.58 µL DIPEA to 5 ml of Fmoc amino acid solution successively, and then adding 700 µL DMF for dissolving; adding the above mixture to the resin mixture; reacting for 1 hour, washing with DMF, CH₂Cl₂ (2x) and DMF successively; and
(5) performing HPLC high performance liquid chromatography to purify and separate the synthesized polypeptides; with conditions: C18 chromatographic column ODS-34.6×250 mm, mobile phase A: H₂O, 0.065% trifluoroacetic acid, mobile phase B: acetonitrile, 0.05% trifluoroacetic acid, flow rate 1 mL/min, gradient conditions: A: 0-25 min, 5%-65%; 25-27 min, 65%-95%; 27-35 min, 95%-5%.
